(19) Europäisches Patentamt
European Patent Office
Office européen des brevets

(11) **EP 4 458 862 A1**

(12) **EUROPEAN PATENT APPLICATION**
published in accordance with Art. 153(4) EPC

(43) Date of publication:
06.11.2024 Bulletin 2024/45

(21) Application number: 21969269.6

(22) Date of filing: 27.12.2021

(51) International Patent Classification (IPC):
C07K 19/00 (2006.01)       C12N 7/01 (2006.01)
C12N 15/62 (2006.01)       C12N 15/863 (2006.01)
A61K 35/76 (2015.01)       A61K 35/768 (2015.01)
A61K 39/395 (2006.01)       A61P 35/00 (2006.01)

(52) Cooperative Patent Classification (CPC):
A61K 35/76; A61K 35/768; A61K 39/395;
A61P 35/00; C07K 19/00; C12N 7/00; C12N 15/62;
C12N 15/863

(86) International application number:
PCT/CN2021/141634

(87) International publication number:
WO 2023/122882 (06.07.2023 Gazette 2023/27)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Shanghai Sinobay Biotechnology Co.,
Ltd.**
**Shanghai 201506 (CN)**

(72) Inventors:
• **XU, Jianqing**
  **Shanghai 201506 (CN)**
• **ZHANG, Xiaoyan**
  **Shanghai 201506 (CN)**

• **DING, Xiangqing**
  **Shanghai 201506 (CN)**
• **LIAO, Qibin**
  **Shanghai 201506 (CN)**
• **WANG, Shiyu**
  **Shanghai 201506 (CN)**
• **ZHOU, Junhua**
  **Shanghai 201506 (CN)**

(74) Representative: **Potter Clarkson
Chapel Quarter
Mount Street
Nottingham NG1 6HQ (GB)**

Remarks:
The complete document including Reference
Table(s) and the Sequence Listing(s) can be
downloaded from the EPO website

(54) **BISPECIFIC T-CELL ENGAGER, RECOMBINANT ONCOLYTIC VIRUS THEREOF, AND USE
THEREOF**

(57)    Provided by the present invention are a bispecific T-cell engager, recombinant oncolytic virus thereof, and use thereof. The present invention provides an αCD47 and αCD3 bispecific T-cell engager. The present invention also provides an isolated nucleic acid molecule that encodes said bispecific T-cell engager. The present invention also provides an expression framework of said bispecific T-cell engager BiTE. The present invention also provides a recombinant oncolytic virus, and said oncolytic virus is operably inserted with or contains the expression framework of said bispecific T-cell engager BiTE. In the present invention, the bispecific T-cell engager is combined with the oncolytic virus, and in comparison with pure gene therapy or virotherapy, the oncolytic virus significantly enhances the inhibition capability on malignant tumors.

**EP 4 458 862 A1**

## SK-OV3

FIG. 2

## Description

## Technical Field

The present invention is in the field of biomedical technology and relates to a novel bispecific T-cell engager (BiTE) comprising human CD47 and human CD3 specific binding fragments. The present invention also relates to a recombinant oncolytic virus comprising the bispecific T-cell engager (BiTE). The present invention further provides a method for preparing the bispecific T-cell engager and the oncolytic virus and the use thereof in antitumor.

## Background Art

Cancer is the second leading cause of death worldwide, accounting for an estimated 9.6 million deaths in 2018. Globally, approximately one sixth deaths are caused by cancer. The situation in China is particularly worrying, in part because of rapid population growth and socio-economic development. Although the incidence of cancer in China is lower than that in developed European and American countries, the cancer mortality rate in China is 30%-40% higher than that in European and American countries, and the prognosis is significantly worse. Nowadays, cancer is getting more and more attention in China, and the related cancer treatment methods, especially tumor immunotherapy with the help of the human body's immune system, are increasingly becoming the focus of attention.

Human CD47, an integrin-associated protein, is a cell membrane surface glycoprotein belonging to the immunoglobulin superfamily. CD47 is widely expressed on the surface of various cancer cells. It releases the "don't eat me" signal by linking to signaling regulatory protein alpha (SIRPα) on the surface of tumor phagocytes, thereby preventing macrophage phagocytosis. In recent years, many companies have invested a lot of manpower and material resources in the research and development of CD47 antibodies, but the results have been minimal after entering the clinic. Currently, the best level of CD47 antibodies is that they can be included in combination drugs. The main reason for the poor efficacy of the treatment is that the side effects are difficult to be controlled, which is attributed to the lethal weakness of CD47, i.e. expression on erythrocytes. When the CD47 antibody drug or SIRPα-Fc fusion protein binds to red blood cells, it can cause red blood cells to agglutinate and then trigger the rupture of red blood cells; the CD47 antibody drug with Fc of IgG1 can also activate phagocytosis of red blood cells by macrophages or antibody-dependent cell-mediated cytotoxicity (ADCC), further trigger lysis of red blood cells and eventually lead to the occurrence of anemia.

How to maximize the use of CD47 targets in killing tumor cells while reducing the damage to red blood cells has been the key to the successful development of CD47-targeted drugs. The bispecific T-cell engager BiTE represents a class of bispecific antibodies with significant anti-tumor effects, which are capable of target-activating autologous T cells to kill tumor cells. BiTE consists of two single-stranded variable fragments (scFv) connected in series by a flexible linker. One scFv recognizes the T cell surface protein CD3εH, while the other scFv recognizes a specific tumor cell surface antigen. This structure of BiTE and the ability to specifically bind proteins allows it to physically bridge T cells to tumor cells to form T cell-BiTE-tumor cell complexes, induce immune synapse formation, stimulate T cell activation, and produce tumor-killing cytokines. In recent years, BiTE has made remarkable progress in anti-tumor research and achieved an ideal therapeutic effect in clinical practice.

Tumor-targeted oncolytic viruses have attracted considerable attention over the last decades due to the limited therapeutic efficacy and severe side effects of standard tumor treatment regimens in the treatment of advanced tumors. Oncolytic viruses are increasingly becoming the first choice for anti-tumor because of their ability to selectively infect and lyse tumor cells locally. The tumor selectivity of an oncolytic virus may be a result of its own tumor tropism or genetic modification. Oncolytic viruses can act on multiple cellular pathways, thereby reducing tumor resistance and also inducing different forms of cell death. In addition, oncolytic viruses can break the immune tolerance of the tumor microenvironment and induce long-term tumor-specific immune responses. Oncolytic viruses can specifically transport therapeutic proteins into tumor tissue with increased expression levels in malignant tumor cells following further viral replication. In addition, oncolytic viruses can be used in combination with chemotherapy, radiotherapy, and immunotherapy.

In 2006, the oncolytic adenovirus product (oncorine) was used for the clinical treatment of nasopharyngeal carcinoma in China. The E1B-55kD gene of human adenovirus type 5 is deleted in this oncolytic virus, which could replicate and proliferate in the cancer cells mutated in the p53 gene and kill the host cells, resulting in the oncolytic therapeutic effect; simultaneous deletion of the E3 region allows the tumor antigen message to activate T cell immunity via dendritic cell transmission. However, clinical data show that oncolytic virus oncorine combined with chemotherapy is less effective than radiotherapy in patients with nasopharyngeal carcinoma.

In 2015, Amgen's oncolytic herpes simplex virus (talimogene laherparepvec, T-VEC) was approved by the US FDA for the treatment of melanoma. In December of the same year, it was approved by the European Union for the local treatment of unresectable skin, subcutaneous, and lymph node lesions in patients with melanoma who relapsed after the first surgery. The results of the T-VEC clinical study have greatly promoted the development of the oncolytic virus in the field of tumor therapy. However, the intratumoral administration method limits the types of treatments and can only be used for tumors close to the body surface that are easy to operate on. There are problems of difficulty in administering drugs and incomplete treatment for many non-superficial solid tumors and metastatic tumors. If it can be confirmed that intravenous administration still has a good tumor treatment effect, its clinical application value will be greatly improved; due to the limited oncolytic effects of herpes viruses themselves, incomplete clearance of bulky and/or metastatic tumors, there is a need to combine other therapeutic approaches to enhance anti-tumor effects.

The biological characteristics and pathogenesis of the vaccinia virus (W) are relatively clear, which plays a key role in the elimination of smallpox, and its safety in the human body has been fully demonstrated. According to the characteristics of pathogenicity and host range, the vaccinia virus can be divided into the WR (Western reserve) strain, Wyeth strain, Copenhagen strain, Lister strain, and Tian Tan strain. Vaccinia virus is used as a vector for multiple recombinant vaccines, such as influenza virus and human immunodeficiency virus, because of its wide host range, high conservation, good safety, and large capacity of exogenous genes. As for the development of oncolytic viruses, most of them are in the preclinical research stage, and only a few of them are in the clinical research stage.

The fastest-progressing research on using vaccinia virus as an oncolytic virus to treat tumors is Pexa-Vec (JX-594) developed by Jennerex, USA. JX-594 is based on the Wyeth strain virus, inserts hGM-CSF and LacZ genes in the TK region, and due to deletion of the thymokinase gene, JX-594 can express and replicate in cancer cells expressing thymokinase at a high level but has no effect on normal cells. At the same time, JX-594 can express GM-CSF in tumor cells by inserting the GM-CSF gene, activating the body's anti-tumor immune response. The clinical trial results of JX-594 in multiple tumor types showed that intratumoral administration or intravenous drip administration had good tolerability, and the effect of Pexa-Vec combined with sorafenib was better than that of a single drug group. The results of the interim analysis showed that it was not likely to prolong the survival of patients. The Phase III clinical trial of Pexa-Vec, which was originally planned to be launched in 2020, was terminated ahead of schedule.

The oncolytic virus (GL-ONC1, also known as GLV-1h68) developed by Genelux, USA, is based on vaccinia virus (Lister strain), deleting its genes F14.5L, TK (encoding thymidine kinase) and HA (encoding hemagglutinin) to enhance tumor targeting, and inserting luciferase-GFP fusion protein, β-galactosyl transferase and β-glucuronidase, respectively, for poxvirus screening and production preparation. The phase I clinical trial of intravenous administration of GL-ONC1 has been completed and showed good safety and efficacy, without dose-limiting toxicity and reaching the maximum tolerated dose, and all patients had neutralization reaction to GL-ONC1; GL-ONC1 is currently undergoing an expanded trial with intravenous administration.

The life cycle of the vaccinia virus is strictly carried out in the cytoplasm of the host cell, and the thymidine kinase gene of the vaccinia virus is required for successful replication of the genome of the progeny virus. However, in the normal tissue cell cycle, the synthesis of cellular Thymidine Kinase (TK) occurs in the S phase of the cell division cycle, after cell division is completed, thymidine kinase is degraded inside the cell, so the concentration of thymidine kinase in the cytoplasm is low. However, the cell division of tumor cells is active, and thymidine kinase is continuously synthesized. Using this feature, the thymidine kinase gene of the vaccinia virus is deleted, resulting in the specific amplification of the vaccinia virus in tumor cells and oncolysis.

Evidence from clinical trials indicates that the vaccinia virus has shown some initial advantages in tumor therapy. Most of the existing designs using vaccinia virus as an oncolytic virus have high safety and clinical efficacy to be further observed, but further optimization is needed in immunomodulation and precise tumor targeting.

Based on the above, there is no bispecific T-cell engager in the prior art that is particularly suitable for recombinant oncolytic viruses, and there is currently a need for safer, more targeted bispecific T-cell engagers and oncolytic viruses comprising them.

**Summary of the Invention**

It is therefore an object of the present invention to address the deficiencies of the prior art to provide a bispecific T-cell engager. The present invention also provides recombinant oncolytic viruses expressing the bispecific T-cell engager (BiTE). The bispecific T-cell engager (BiTE) provided by the present invention overcomes the limitation of solid tumor cells to human T cells in tumor immune editing (immunosuppression and immune evasion), and can directly recruit human T cells to the interior of solid tumors for tumor cell killing, with broad application prospects.

The objects of the present invention are achieved by the following technical solutions:

In one aspect, the invention provides an $\alpha$CD47 and $\alpha$CD3 bispecific T-cell engager $\alpha$CD47-$\alpha$CD3 BiTE, comprising a fusion protein of any one of the following formulae:

$$VL_{\alpha CD47}\text{-}L\text{-}VH_{\alpha CD3}\text{-}L\text{-}VL_{\alpha CD3}\text{-}L\text{-}VH_{\alpha CD47};$$

or

$$VH_{\alpha CD47}\text{-}L\text{-}VH_{\alpha CD3}\text{-}L\text{-}VL_{\alpha CD3}\text{-}L\text{-}VL_{\alpha CD47}$$

wherein the $VH_{\alpha CD47}$ is the heavy chain variable region of a CD47 antibody comprising the following three complementary determining regions:

(i) VH CDR1 consisting of the following sequence: SEQ ID NO: 1, or a sequence having one or several amino acid substitutions, deletions, or additions compared thereto,

(ii) VH CDR2 consisting of the following sequence: SEQ ID NO: 2, or a sequence having one or several amino acid substitutions, deletions, or additions compared thereto, and

(iii) VH CDR3 consisting of the following sequence: SEQ ID NO: 3, or a sequence having one or several amino acid substitutions, deletions, or additions compared thereto;

preferably, the substitution of any one of (i)-(iii) is a conservative substitution;

preferably, the VH$_{\alpha CD47}$ comprises VH CDR1 as shown in SEQ ID NO: 1, VH CDR2 as shown in SEQ ID NO: 2, VH CDR3 as shown in SEQ ID NO: 3;

wherein the VL$_{\alpha CD47}$ is the light chain variable region of a CD47 antibody comprising the following three complementary determining regions:

(iv) VL CDR1 consisting of the following sequence: a sequence as shown in any one of SEQ ID NO: 4, SEQ ID NO: 7, SEQ ID NO: 10, SEQ ID NO: 13 or SEQ ID NO: 16, or having one or several amino acid substitutions, deletions, or additions compared thereto,

(v) VL CDR2 consisting of the following sequence: a sequence as shown in any one of SEQ ID NO: 5, SEQ ID NO: 8, SEQ ID NO: 11, SEQ ID NO: 14, or SEQ ID NO: 17, or having one or several amino acid substitutions, deletions, or additions compared thereto, and

(vi) VL CDR3 consisting of the following sequence: a sequence as shown in any one of SEQ ID NO: 6, SEQ ID NO: 9, SEQ ID NO: 12, SEQ ID NO: 15, or SEQ ID NO: 18, or having one or several amino acid substitutions, deletions, or additions compared thereto;

preferably, the substitution of any one of (iv)-(vi) is a conservative substitution;

preferably, the VL$_{\alpha CD47}$ comprises VL CDR1 as shown in SEQ ID NO: 4, VL CDR2 as shown in SEQ ID NO: 5, and VL CDR3 as shown in SEQ ID NO: 6;

or the VL$_{\alpha CD47}$ comprises VL CDR1 as shown in SEQ ID NO: 7, VL CDR2 as shown in SEQ ID NO: 8, and VL CDR3 as shown in SEQ ID NO: 9;

or the VL$_{\alpha CD47}$ comprises VL CDR1 as shown in SEQ ID NO: 10, VL CDR2 as shown in SEQ ID NO: 11, and VL CDR3 as shown in SEQ ID NO: 12;

or the VL$_{\alpha CD47}$ comprises VL CDR1 as shown in SEQ ID NO: 13, VL CDR2 as shown in SEQ ID NO: 14, and VL CDR3 as shown in SEQ ID NO: 15;

or the VL$_{\alpha CD47}$ comprises VL CDR1 as shown in SEQ ID NO: 16, VL CDR2 as shown in SEQ ID NO: 17, and VL CDR3 as shown in SEQ ID NO: 18.

In the bispecific T-cell engager according to the invention, the VH$_{\alpha CD47}$ comprises three CDRs contained in the heavy chain variable region as shown in SEQ ID NO: 19;

preferably, the VL$_{\alpha CD47}$ comprises three CDRs contained in the light chain variable region as shown in any one of SEQ ID NOs: 20-24; and

preferably, the three CDRs contained in the heavy chain variable region and/or the three CDRs contained in the light chain variable region are defined by the Kabat, Chothia, or IMGT numbering system.

In the bispecific T-cell engager according to the invention, the VH$_{\alpha CD47}$ comprises an amino acid sequence selected from the group consisting of:

(i) a sequence as shown in SEQ ID NO: 19;

(ii) a sequence having one or several amino acid substitutions, deletions, or additions compared to the sequence as shown in SEQ ID NO: 19; or

(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 19; and/or,

the VL$_{\alpha CD47}$ comprises an amino acid sequence selected from the group consisting of:

(iv) a sequence as shown in any one of SEQ ID NOs: 20-24;

(v) a sequence having one or several amino acid substitutions, deletions, or additions compared to the sequence as shown in any one of SEQ ID NOs: 20-24; or

(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in any one of SEQ ID NOs: 20-24; and

preferably, the substitution in (ii) or (v) is a conservative substitution.

In the bispecific T-cell engager according to the invention, the VH$_{\alpha CD3}$ comprises an amino acid sequence selected from the group consisting of:

(i) a sequence as shown in SEQ ID NO: 25 or 27;

(ii) a sequence having one or several amino acid substitutions, deletions, or additions compared to the sequence as shown in SEQ ID NO: 25 or 27; or

(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 25 or 27; and/or,

the $VL_{\alpha CD3}$ comprises an amino acid sequence selected from the group consisting of:

(iv) a sequence as shown in SEQ ID NO: 26 or 28;

(v) a sequence having one or several amino acid substitutions, deletions, or additions compared to the sequence as shown in SEQ ID NO: 26 or 28; or

(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 26 or 28; and

preferably, the substitution in (ii) or (v) is a conservative substitution.

In the bispecific T-cell engager according to the invention, the $VH_{\alpha CD47}$, $VH_{\alpha CD3}$, $VL_{\alpha CD47}$, and $VL_{\alpha CD3}$ are linked by a linker peptide;

preferably, the $VH_{\alpha CD47}$, $VH_{\alpha CD3}$, $VL_{\alpha CD47}$, and $VL_{\alpha CD3}$ are optionally linked by one, two, or three linker peptides;

preferably, the bispecific T-cell engager comprises a fusion protein according to any one of the following formulae:

$$VL_{\alpha CD47}\text{-}L\text{-}VH_{\alpha CD3}\text{-}L\text{-}VL_{\alpha CD3}\text{-}L\text{-}VH_{\alpha CD47};$$

or

$$VH_{\alpha CD47}\text{-}L\text{-}VH_{\alpha CD3}\text{-}L\text{-}VL_{\alpha CD3}\text{-}L\text{-}VL_{\alpha CD47}$$

more preferably, L is KESGSVSSEQLAQFRSLD, EGKSSGSGSESKST, GGGGGG, GGGGGGGG or $(GGGGS)_n$; further preferably, n is an integer from 1 to 5, most preferably n is 3; and

preferably, the bispecific T-cell engager comprises a fusion protein of the following formula:

$$VH_{\alpha CD47}\text{-}L\text{-}VH_{\alpha CD3}\text{-}L\text{-}VL_{\alpha CD3}\text{-}L\text{-}VL_{\alpha CD47}$$

wherein the $VH_{\alpha CD47}$ is as shown in SEQ ID NO: 19, the $VL_{\alpha CD47}$ is as shown in SEQ ID NO: 21 or 24, the $VH_{\alpha CD3}$ is as shown in SEQ ID NO: 25, $VL_{\alpha CD3}$ is as shown in SEQ ID NO: 26, and the L is GGGGSGGGGSGGGGS.

The present invention also provides an isolated nucleic acid molecule that encodes the bispecific T-cell engager;

preferably, the nucleic acid molecule comprises a nucleic acid sequence as shown in any one of SEQ ID NOs: 31-35, the nucleic acid sequence is the coding sequence for $VL_{\alpha CD47}$; and

preferably, the nucleic acid molecule comprises a nucleic acid sequence as shown in SEQ ID NO: 36, the nucleic acid sequence is the coding sequence for $VH_{\alpha CD47}$.

An expression framework for the bispecific T-cell engager according to the invention: 5 '-E1-E2-E3 -E4-E5 -E6-E7-3'
wherein:

E1 is the CMV enhancer or/and other cis-acting elements, preferably comprises the nucleic acid sequence as shown in SEQ ID NO: 37;

E2 is a recombinantly expressed promoter, preferably a CMV promoter, more preferably comprising the nucleic acid sequence as shown in SEQ ID NO: 38;

E3 is a 5' untranslated region, optionally comprising no or one intron sequence, optionally comprising one or more restriction enzyme sites, preferably comprises the nucleic acid sequence as shown in SEQ ID NO: 39;

E4 is a coding nucleotide sequence of the signal peptide of the BiTE protein; preferably, the signal peptide is derived from a human or mouse signal peptide; preferably E4 comprises the nucleic acid sequence as shown in SEQ ID NO: 40;

E5 is the coding nucleotide sequence of the bispecific T-cell engager;

E6 is a 3' untranslated region, optionally comprising one or more restriction enzyme sitespreferably comprises the nucleic acid sequence as shown in SEQ ID NO: 41; and

E7 is the SV40 transcription termination signal region, preferably comprising the nucleic acid sequence as shown in SEQ ID NO: 42.

In yet another aspect, the present invention provides a recombinant oncolytic virus, and the oncolytic virus is operably inserted with or contains the expression framework of the bispecific T-cell engager BiTE.

Preferably, the expression framework is located in the thymidine kinase (TK) region of the recombinant oncolytic virus.

Preferably, the expression framework can be expressed alone, and can also be fusion expressed with other genes or fragments.

Preferably, the recombinant oncolytic virus further comprises gene coding sequences for other immunomodulatory factors, more preferably, the other immunomodulatory factors include but are not limited to IL-1, IL-2, IL-3, IL-7, IL-11, IL-12, IL-15, IL-17, IL-18, IL-21, IL-33, IL-35, IL-37, GM-CSF, IFN-$\alpha$, IFN-$\beta$, IFN-$\gamma$, anti-PD-1/PD-L1 antibody, anti-CTLA-4 antibody, anti-Lag-3 antibody, anti-TIGIT antibody or anti-Tim-3 antibody; or

the recombinant oncolytic virus further comprises gene coding sequences of apoptosis- and pyroptosis-related proteins, including but not limited to apoptosis-related factor 1 (Apaf-1), interleukin-1β converting enzyme (ICE), Bcl-2 protein, Fas/APO-1, p53, myc, ataxia telangiectasia mutant gene (ATM), gasdermin D, gasdermin E, etc.; or the recombinant oncolytic virus further comprises a small RNA targeting immunomodulatory genes, apoptosis and pyroptosis genes.

In the recombinant oncolytic virus according to the present invention, the viral backbone of the oncolytic virus is derived from a modified or engineered vaccinia virus Tian Tan strain, vaccinia virus New York strain, vaccinia virus Copenhagen strain, vaccinia virus canary strain, vaccinia virus Ankara strain, adenovirus, adeno-associated virus, herpes simplex virus, varicella-zoster virus (VZV), respiratory syncytial virus (RSV), Semliki forest virus (SFV), EB virus, cytomegalovirus, human Herpesvirus type 6, smallpox virus, vaccinia virus, molluscum contagiosum virus, sheep aphthovirus, reovirus, rotavirus, enterovirus, Seneca virus, poliovirus, coxsackie virus, rhinovirus, hepatitis A virus, foot and mouth disease virus, togavirus, alphavirus, Semiliki forest virus, eastern equine encephalitis virus, Sindbis virus, rubella virus, coronavirus, flavivirus, hepatitis C virus, Japanese encephalitis virus, St. Louis encephalitis virus, Murray Valley fever virus, yellow fever virus, West Nile virus, zika virus, dengue virus, Ebola virus, Marburg virus, arenavirus, Lassa fever virus, lymphocytic choriomeningitis virus, Pichinde virus, Junin virus, Machupo virus, Hantavirus, Rift Valley fever virus, Paramyxovirus, human parainfluenza virus, Mumps virus, simian virus 5, measles virus, vesicular stomatitis virus, rabies virus, orthomyxovirus, influenza A virus, influenza B virus, influenza C virus, hepatitis D virus, simian immunodeficiency virus, human immunodeficiency virus type 1 and human immunodeficiency virus type 2, Rous sarcoma virus, human T-cell leukemia virus type 1, simian foamy virus, hepatitis B virus, hepatitis E virus, human papilloma virus, or polyomavirus.

Preferably, the oncolytic viral backbone is an intracellular maturation virus, an intracellular packaging virus, a cell-associated packaging virus, or an extracellular packaging virus.

The recombinant oncolytic virus according to the present invention is a recombinant vaccinia virus Tian Tan strain comprising a gene encoding the bispecific T-cell engager αCD47-αCD3-BITE, named rTV-αCD47-αCD3-BITE, with a deposit accession number of CCTCC NO: V202081, a deposit date of January 2, 2021, and a depositary institution of China Center for Type Culture Collection.

In yet another aspect, the present invention provides a method for preparing the recombinant oncolytic virus, comprising the steps of:

1) synthesizing an expression framework of the bispecific T-cell engager BiTE;
2) subcloning the expression framework obtained in step 1) into a shuttle plasmid of an oncolytic virus to construct a recombinant plasmid vector;
3) transfecting the recombinant plasmid vector obtained in step 2) into an oncolytic virus, and creening the same so as to obtain a recombinant oncolytic virus;

optionally, culturing the obtained recombinant oncolytic virus.

In a specific embodiment, the present invention provides a method for preparing a recombinant vaccinia virus Tian Tan strain, comprising the steps of:

1) synthesizing an expression framework of the bispecific T-cell engager αCD47-αCD3-BITE, which comprises the nucleic acid sequence as shown in any one of SEQ ID NOs: 31-42;
2) subcloning the synthesized expression framework into the TK region of the vaccinia virus shuttle plasmid (pSC65) to construct the recombinant plasmid pSC65-αCD47-αCD3-BITE;
3) transfecting the pSC65-αCD47-αCD3-BITE plasmid into TK143-cells which have been infected with wild-type vaccinia virus by means of gene homologous recombination, and homologously recombing the two to produce recombinant vaccinia virus rTV-αCD47-αCD3-BITE; screening the same to obtain a recombinant oncolytic vaccinia virus wherein the TK region comprises the coding sequence of αCD47-αCD3-BITE;

wherein, the expression framework of the bispecific T-cell engager αCD47-αCD3-BITE is controlled by the early/-late promoter p7.5 of vaccinia virus.

Preferably, amplifying the recombinant vaccinia virus using VERO cells comprises the specific steps of: replacing the medium with the low-concentration fetal bovine serum maintenance medium when VERO cells are cultured to a density close to 100%, adding oncolytic vaccinia virus (the inoculation amount is about 0.02 MOI per 10 cm culture plate), and placing the same into an incubator for culture, collecting the virus solution after completion of amplification of recombinant poxvirus, freeze-thawing the same repeatedly, and then purifying the same by density gradient centrifugation using sucrose solution.

In a further aspect, the present invention also provides use of the bispecific T-cell engager or recombinant oncolytic virus for the preparation of an anti-tumour medicament; wherein the tumor is selected from the group consisting of B-cell lymphoma; T-cell lymphoma; melanoma; prostate cancer; renal cell carcinoma; sarcoma; glioma, such as high-grade glioma; blastoma, such as neuroblastoma; osteosarcoma; plasmacytoma; histiocytoma; pancreatic cancer; breast cancer; lung cancer, such as small cell lung cancer and non-small cell lung cancer; gastric cancer; liver cancer; colon cancer; rectal cancer; esophageal cancer; colorectal cancer; hematopoietic cancer; testicular cancer; cervical cancer; ovarian cancer; bladder cancer; squamous cell cancer; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or hematological tumorigenesis disease.

In another aspect, the invention provides a method of treating a tumor, comprising administering to a subject in need thereof a therapeutically effective amount of a bispecific T-cell engager or a recombinant oncolytic virus. The method according to the present invention further comprises administering to the subject in need thereof an additional chemotherapeutic agent, a radiation therapy technique, a surgical treatment, an immune cell drug (including but not limited to CAR-T, NK, NKT, iNKT, CAR-NK, CAR-NKT, CAR-iNKT, etc.), other oncolytic viruses; preferably, the method is intravenous injection or intratumoral injection.

The inventive concept of the present invention is as follows:

The present invention provides a novel αCD47-αCD3-BITE bispecific T-cell engager with good anti-tumor activity in vitro. Further, the present inventors have provided a vaccinia virus Tian Tan strain carrying the bispecific T-cell engager of the present invention on the basis of a research mode of vaccinia virus Tian Tan strain. Then, the inventors of the present invention found that the vaccinia virus Tian Tan strain itself has a strong anti-tumor effect; the bispecific T-cell engager carrying αCD47-αCD3-BITE can avoid the binding of CD47 antibody to the red blood cells of peripheral blood, recognize tumor cells by using the broad-spectrum expression characteristics of CD47, and activate T cells by CD3 antibody, thereby playing the role of killing tumor cells, improving the effect of tumor immunotherapy and increasing safety.

Advantageous effects of the present invention are:

1. The bispecific T-cell engager (BiTE) provided by the present invention has a small molecular weight, so it is capable of penetrating the intercellular space to reach the tumor microenvironment (TME) inside solid tumors, thereby being capable of efficiently mediating the killing of cancer cells by T cells.

2. The present invention combines a bispecific T-cell engager with an oncolytic virus to provide a vaccinia virus Tian Tan strain highly expressing the bispecific T-cell engager αCD47-αCD3-BITE gene. When the oncolytic virus is administered to tumor lesions, it lyses tumor cells to exert an oncolytic effect, and at the same time carries a tumor-targeted gene high-killing antibody against human CD47, blocks the binding of CD47 to SIRPα, and promotes macrophage phagocytosis in vivo, so as to enhance the anti-tumor effect. The oncolytic virus significantly enhances the ability to inhibit malignant tumors when compared to gene therapy or virotherapy alone.

3. According to the in vitro experimental study of the present invention, it is proved that the vaccinia virus Tian Tan strain of the present invention can treat liver cancer and malignant lung cancer, achieve good targeting to tumors and anti-tumor effect, have a relatively complete virus amplification and quality control system, and lay the foundation for further industrialization, and therefore, the present invention has a good application prospect.

**Brief Description of the Drawings**

Hereinafter, embodiments of the present invention will be described in detail with reference to the accompanying drawings, in which:

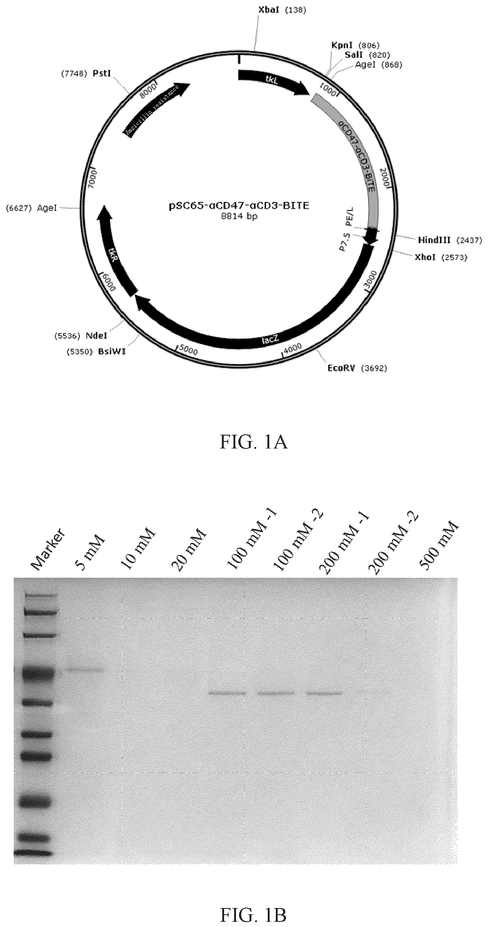

FIG. 1 shows the construction of the shuttle plasmid vector αCD47-αCD3 BiTE hIgG1 WT of the present invention and the expression and purification of five corresponding BiTE proteins; wherein, FIG. 1A is an expression map of shuttle plasmid hIgG1 WT integrated with the αCD47-αCD3-BITE expression framework; FIG. 1B is the result of supernatants of medium with 293T transiently expressing αCD47-αCD3-BITE after protein purification using a nickel column.

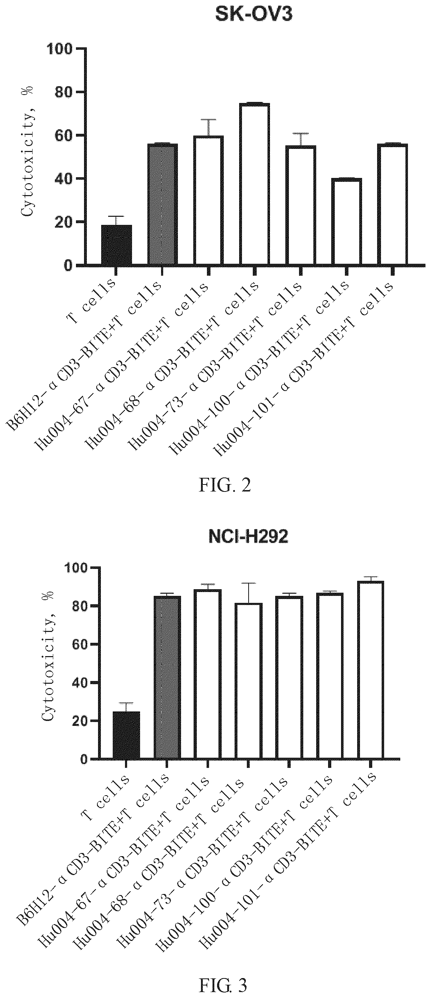

FIG. 2 shows the effect of αCD47-αCD3-BITE mediated T cells in vitro against human ovarian adenocarcinoma cells; wherein, compared with the T cell control group, the BiTE composed of the CD47 antibody of clone No. 68 and αCD3 can well mediate the killing of SK-OV3 by T cells. The results show that αCD47-αCD3-BITE can recognize CD47 tumor cells and activate T cells, thereby mediating the specific killing of the tumor cells by T cells; it has been shown that a bispecific T-cell engager (BITE) comprising specific binding of human CD47 and human CD3 mediates T cell antitumor activity well in vitro and has a broad spectrum of properties.

FIG. 3 shows the effect of αCD47-αCD3-BITE mediated T cells in vitro against lung cancer cells; wherein, compared with the T cell control group, BiTE composed of CD47 antibody of clone No. 101 and αCD3 can well mediate the killing of NCI-H292 tumor cells by T cells. Compared with the T cell alone group, the BiTE group has an obvious killing effect on NCI-H292 cells; it has been shown that a bispecific T-cell engager (BITE) comprising specific binding of human CD47 and human CD3 mediates T cell antitumor activity well in vitro and has a broad spectrum of properties.

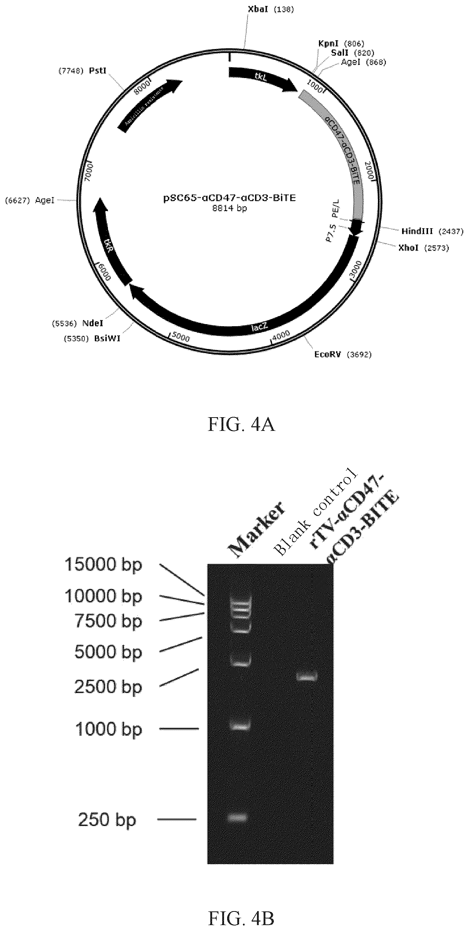

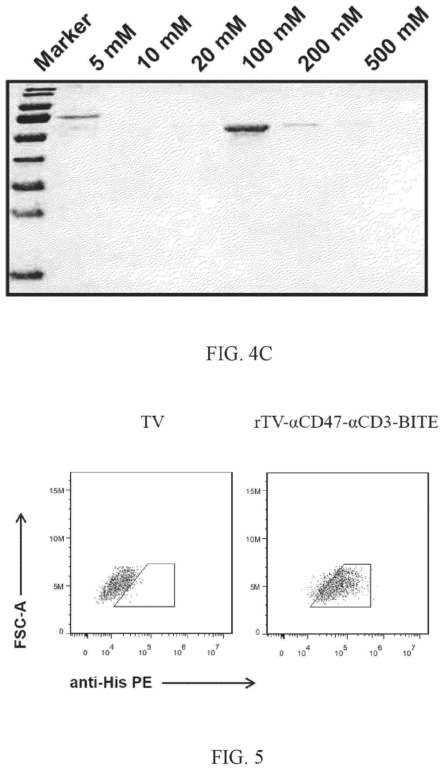

FIG. 4 shows the construction of the vaccinia virus Tian Tan strain shuttle plasmid vector ($\alpha$CD47-$\alpha$CD3-BITE) and the expression of $\alpha$CD47-$\alpha$CD3-BITE protein. FIG. 4A is an expression map of vaccinia virus shuttle plasmid pSC65 integrated with $\alpha$CD47-$\alpha$CD3-BITE gene; FIG. 4B is the results of PCR identification of the insert fragment $\alpha$CD47-$\alpha$CD3-BITE in the recombinant vaccinia virus rTV-$\alpha$CD47-$\alpha$CD3-BITE; FIG. 4C shows CD47-$\alpha$CD3-BITE protein expression in supernatants of recombinant vaccinia virus-infected VERO cells. As can be seen from FIG. 4, $\alpha$CD47-$\alpha$CD3-BITE carried in the recombinant vaccinia virus rTV-$\alpha$CD47-$\alpha$CD3-BITE was successfully expressed.

FIG. 5 shows that the $\alpha$CD47-$\alpha$CD3-BITE carried by the recombinant vaccinia virus rTV-$\alpha$CD47-$\alpha$CD3-BITE has a high affinity for the CD47 protein; FIG. 5 is the flow cytometry results of affinity assay of cell supernatant collected from VERO cells infected with recombinant vaccinia virus rTV-$\alpha$CD47-$\alpha$CD3-BITE with lung cancer cells A549 expressing human CD47; the results show that compared with wild-type vaccinia virus, the protein expressed by the recombinant vaccinia virus rTV-$\alpha$CD47-$\alpha$CD3-BITE has more than 85% binding rate to CD47.

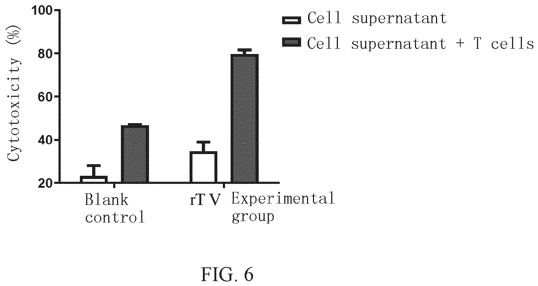

FIG. 6 shows the killing effect of recombinant vaccinia virus of vaccinia virus Tian Tan strain rTV-$\alpha$CD47-$\alpha$CD3-BITE on A549 cells in vitro. The experiment was divided into four groups, including a wild vaccinia virus-infected supernatant control group, a wild vaccinia virus-infected supernatant + T cell group, a recombinant vaccinia virus-infected supernatant control group, and a recombinant vaccinia virus-infected supernatant + T cell group. It can be seen from the figure that compared with the blank control group, the recombinant vaccinia virus-infected supernatant + T cell group has a significant killing effect on lung cancer cells A549 cells.

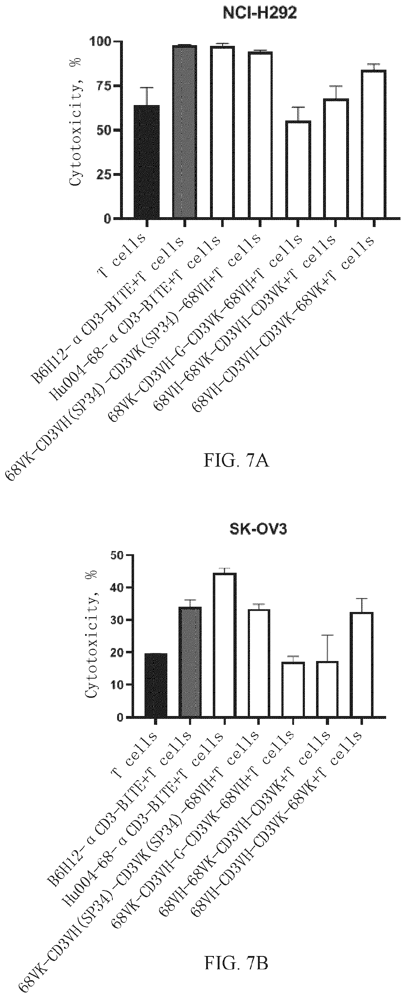

FIG. 7 shows the anticancer effects of $\alpha$CD47-$\alpha$CD3-BITE proteins of different structures.

## Deposit information

The vaccinia virus Tian Tan strain is named rTV-$\alpha$CD47-$\alpha$CD3-BITE and has the deposit accession number of CCTCC NO: V202081, the deposit date of January 2, 2021, and the depositary institution of China Center for Type Culture Collection (address: Wuhan University, Wuhan, China).

## Mode of Carrying Out the Invention

Example 1: Construction and expression verification of 293T recombinantly expressed plasmid of $\alpha$CD47-$\alpha$CD3-BITE

### 1.1 Construction of hIgG1 WT vector with $\alpha$CD47-$\alpha$CD3-BITE target gene

First, five high affinity anti-human CD47 antibodies were screened, their heavy chain and light chain variable regions were selected, and a fusion protein was constructed according to the following formula: $VH_{\alpha CD47}$-L-$VH_{\alpha CD3}$-L-$VL_{\alpha CD3}$-L-$VL_{\alpha CD47}$; wherein the $VH_{\alpha CD47}$ was as shown in SEQ ID NO: 19, the $VL_{\alpha CD47}$ was as shown in any one of SEQ ID NOs: 20-24, the $VH_{\alpha CD3}$ was as shown in SEQ ID NO: 25, $VL_{\alpha CD3}$ was as shown in SEQ ID NO: 26, and the L was GGGGSGGGGSGGGGS.

Also, depending on $VL_{\alpha CD47}$, Hu004-67 denoted an $\alpha$CD47-$\alpha$CD3 BiTE including $VL_{\alpha CD47}$ as shown in SEQ ID NO: 20; Hu004-68 denoted $\alpha$CD47-$\alpha$CD3 BiTE comprising $VL_{\alpha CD47}$ as shown in SEQ ID NO: 21; Hu004-73 denoted $\alpha$CD47-$\alpha$CD3 BiTE comprising $VL_{\alpha CD47}$ as shown in SEQ ID NO: 22; Hu004-100 denoted $\alpha$CD47-$\alpha$CD3 BiTE comprising $VL_{\alpha CD47}$ as shown in SEQ ID NO: 23; Hu004-101 denoted $\alpha$CD47-$\alpha$CD3 BiTE comprising $VL_{\alpha CD47}$ as shown in SEQ ID NO: 24, wherein WT Cloning vector AbVec-hIgG1 (GenBank: FJ475055.1).

The DNA sequence of $\alpha$CD47-$\alpha$CD3-BITE was artificially synthesized according to the structural formula, and the plasmid construct map was shown in FIG. 1a, wherein the coding sequence for $VL_{\alpha CD47}$ was shown in any one of SEQ ID NOs: 31-35, and the coding sequence for $VH_{\alpha CD47}$ was shown in SEQ ID NO: 36.

The synthetic hIgG1 WT vector containing the target gene $\alpha$CD47-$\alpha$CD3-BITE was transformed into E. coli TOP10 (Weidi, Cat. No. DL1010S) and grown overnight on plates containing ampicillin. On day 2, single colonies were randomly picked for overnight culture at 37°C and plasmids were extracted for 293T cell transfection using a plasmid extraction kit (Qiagen, Cat. No. 27106).

### 1.2 Expression purification of 293T cells with $\alpha$CD47-$\alpha$CD3-BITE target gene

1. 293T cell preparation: the supernatant of the cells growing in T175 culture flask with the confluence exceeding 90% was removed, 2 ml of 2.5% Trypsin-EDTA (GIBCO, Cat. No. 25200072) was added to digest the cells until the cells were completely detached, 10 ml of complete medium (DMEM medium + 10% FBS + 1% PS) was added to stop the digestion, the mixture was centrifuged at 150 g for 5 min to collect the cells, the supernatant was discarded, and the cells were reselected with proper amount of complete medium to make the cell concentration about $8 \times 10^6 \sim 10 \times 10^6$ cells/ml. The cell suspension was counted and $1.3 \times 10^7$ cells were plated in a 150 mm cell culture dish (Thermo Scientific, Cat. No. 150468) with a medium volume of 25 mL and cultured until the next day for subsequent plasmid transfection.

2. Transfecting 293T cells by hIgG1 WT shuttle plasmid of $\alpha$CD47-$\alpha$CD3-BITE: 1 mL DMEM culture medium was taken and put into an EP tube, 20 $\mu$g of the hIgG1 WT shuttle plasmid Cloning vector AbVec-hIgG1 (GenBank: FJ475055.1) of $\alpha$CD47-$\alpha$CD3-BITE was added to mix well, and then 30 $\mu$l FectoPro transfection reagent (Polyplus, Cat. No. 116-001) was added, after that, the mixture was placed on a vortex oscillator immediately and shake vigorously for 15 s, and let it stand at room temperature for 20 min. The 293T cells to be transfected were taken out from the incubator, 10 mL of culture medium was sucked and discarded, the transfection reagent after standing was added while shaking, and the mixture was placed in an incubator containing 5% $CO_2$ at 37°C for 4-6 h. The medium was completely removed and Expi293 expression medium (GIBCO, Cat. No. A1435102) was added and incubated at 37°C in an incubator containing 5% $CO_2$ for 4 days.

3. Collecting the $\alpha$CD47-$\alpha$CD3-BITE expression supernatant: medium was harvested from the expression dishes into a 50 mL centrifuge tube and centrifuged at 4000 g for 5 min and the expression supernatant was transferred to a new 50 mL centrifuge tube.

4. Subjecting the expression supernatant to His tag protein purification: purification was performed using a gravity chromatography column packed with Ni-NTA Agarose (QIAGEN, Cat. No. 30210); after the completion of column loading, the non-specifically adsorbed proteins were eluted with 5 mM and 10 mM imidazole (dissolved in 50 mM $NaH_2PO_4 \cdot 2H_2O$, pH 8.0, 300 mM NaCl) at natural flow rate, and the filtrate was collected; the target protein was then eluted with 20 mM, 100 mM (collecting two tubes in sequence), 200 mM (collecting two tubes in sequence), 500 mM imidazole (dissolved in 50 mM $NaH_2PO_4 \cdot 2H_2O$, pH 8.0, 300 mM NaCl) and the corresponding filtrate was collected.

5. 20 $\mu$L of the filtrate was taken to prepare samples and 10% SDS-PAGE detection was performed. As shown in the figure, samples were loaded sequentially from 5 mM imidazole eluate to 500 mM imidazole eluate. The protein of interest was present in the 100 mM eluate and 200 mM shown in FIG. 1B, with a band size of around 54 KD.

Example 2: Effect of $\alpha$CD47-$\alpha$CD3-BITE mediated T cells in vitro against human ovarian adenocarcinoma cells

1. SK-OV3 cell plating: SK-OV3 (deposited in Shanghai Sinobay Biotech Co. Ltd.) was a Luciferase-expressing cell line. Cells were plated in 96-well plates at a density of $1 \times 10^4$ cells /well and incubated overnight at 37°C for attachment.

2. After 24 h, the medium was removed, and seven groups were set, and T cells, B6H12-$\alpha$CD3-BITE (heavy chain variable region and light chain variable region of B6H12 were shown in SEQ ID NO: 29 and 30, respectively, and the remaining structures were the same as those of the BITE of Example 1 of the present invention) and T cells, Hu004-67-$\alpha$CD3-BITE and T cells, Hu004-68-$\alpha$CD3-BITE and T cells, Hu004-73-$\alpha$CD3-BITE and T cells, Hu004-100-$\alpha$CD3-BITE and T cells, Hu004-101-$\alpha$CD3-BITE and T cells were added, respectively.

3. Overnight culture was performed at 37°C for killing. After 24 h, the supernatant was removed and 50 $\mu$L of cell lysate Luciferase cell lysate (Promega Cat. No. E1531) was added per well and incubated for 30 min at room temperature with shaking and 30 $\mu$L of Luciferase substrate (Promega, Cat. No. E151A) was added per well. On-machine detection (GloMax Navigator Microplate Luminometer, Promega, Steady-Glo protocol) was conducted.

The detected results were shown in FIG. 2. B6H12 was a known CD47 antibody clone (US 9017675B2) as a positive control. Hu004-68 and Hu004-101 were selected antibody clones with high affinity for CD47 protein (obtained by antibody screening under entrusted to Osaka Biotech). As can be seen from FIG. 2, against this tumor cell line, the CD47 antibody (scFv)-$\alpha$CD3-BITE of clone No. 68 showed a 3.5-fold increase in tumor killing activity compared with the T cell control group, a 2-fold increase compared with clone No. 100 with low killing ability, and a 1.5-fold increase compared with the positive control antibody, which can well mediate the in vitro anti-ovarian adenocarcinoma cell effect of T cells.

Example 3: Effect of $\alpha$CD47-$\alpha$CD3-BITE Mediated T Cells in Vitro Against Human Lung Cancer Cells

1. NCI-H292 cell plating: NCI-H292 (deposited in Shanghai Sinobay Biotech Co. Ltd.) was a Luciferase-expressing cell line. Cells were plated in 96-well plates at a density of $1 \times 10^4$ cells /well and incubated overnight at 37°C for attachment.

2. After 24 h, the medium was removed, and seven groups were set, and T cells, B6H12-αCD3-BITE and T cells, Hu004-67-αCD3-BITE and T cells, Hu004-68-αCD3-BITE and T cells, Hu004-73-αCD3-BITE and T cells, Hu004-100-αCD3-BITE and T cells, Hu004-101-αCD3-BITE and T cells were added, respectively.

3. Overnight culture was performed at 37°C for killing. After 24 h, the supernatant was removed and 50 μL of cell lysate (Promega, Cat. No. E1531) was added per well and incubated for 30 min at room temperature with shaking and 30 μL of Luciferase substrate (Promega, Cat. No. E151A) was added per well. On-machine detection (GloMax Navigator Microplate Luminometer, Promega, Steady-Glo protocol) was conducted.

The obtained result was shown in FIG. 3, the CD47 antibody-αCD3-BITE of clone No. 101 can well mediate the anti-lung cancer cell effect of T cells in vitro. The CD47 antibody (scFv)-αCD3-BITE of clone No. 101 had a 4-fold increase in tumor killing activity compared with the T cell control group, a 1.2-fold increase compared with clone No. 100 with low killing ability, and a 1.2-fold increase compared with the positive control antibody, which can well mediate the anti-ovarian adenocarcinoma cell effect of T cells in vitro. Together, the results of FIG. 2 and FIG. 3 demonstrated that a bispecific T-cell engager (BITE) comprising specific binding of human CD47 and human CD3 was able to well mediate T cell antitumor activity in vitro and had a broad-spectrum property.

Example 4: Construction and Expression Verification of Recombinant Vaccinia Virus of αCD47-αCD3-BITE

4.1 Construction of pSC65 vector with αCD47-αCD3-BITE target gene

The DNA sequence of αCD47-αCD3-BITE was artificially synthesized and PCR amplification was performed using the synthesized DNA sequence as a template and using the following primers.
The primers used for amplification were:

αCD47-αCD3-BITE-F: **SEQ ID NO: 43** GTAC-CAGGCCTAGTACTATGGAGAG-GACCCTTGTCTG
αCD47-αCD3-BITE-R: **SEQ ID NO: 44** AA-TAAGCTCGAAGTCGAC CTAGGAGAGATGCT-GATG

PCR reaction procedure: pre-denaturating at 94°C for 5 min; denaturing at 98°C for 10 seconds, 58°C: annealing for 30 seconds, extending at 72°C for 1 min, and reacting for 30 cycles; sufficiently extending at 72°C for additional 10 min, ending at 25°C.
Recovery and cloning of PCR products: after amplification, the target gene was separated on a 2% agarose gel, and the pSC65 vector was linearized by Sal I digestion (Thermo Scientific, Cat. No. ER0642). The digestion gel was recovered, and the PCR fragment and vector enzyme section were recovered using a Sanprep column DNA gel recovery kit (Promega, Cat. No. A9282). The recovered gene product was linked to the digested linearized vector by homologous recombination method (Vazyme, Cat. No. c112-02). The linking product was transformed into E. coli TOP10 and grown overnight on plates containing ampicillin. On Day 2, a single colony was randomly picked for sequencing, and mutation site was corrected, and after verifying that all sequences were correct, the shuttle plasmid pSC65 of αCD47-αCD3-BITE was successfully cloned, and the plasmid construction map was shown in FIG. 4A.

4.2 Construction of recombinant vaccinia virus αCD47-αCD3-BITE

1. Cell preparation: 143TK-cells were plated in 6-well plates at approximately $1 \times 10^6$ cells per well. After incubated for approximately 24 h, the cells attached and spreaded across the bottom, and then the next step was proceeded.
2. Vaccinia virus incubation: 0.0125/3 PFU (PFU: plaque-forming units, virus titer)/cell of wild-type vaccinia virus Tian Tan strain was used to infect the cells. The cells were incubated in an incubator at 37°C for 1 h and then removed. The supernatant was aspirated and washed once with 1 mL PBS and 1 mL of complete medium was added.
3. Plasmid transfection: the shuttle plasmid PSC65-αCD47-αCD3-BITE described above was transfected into 143TK-cells, and incubated at 37°C for about 48 h, depending on the cytopathic effect.
4. 2x DMEM maintenance medium (containing 2% PS and 4% FBS) for virus plating was prepared and 2% pre-warmed low melting agarose was added followed by X-gal (final concentration 200 μg/mL).
5. The supernatant was aspirated from the 6-well plate and the mixture for plating was added to the 6-well plate at 1 mL per well. Then the mixture was carefully put into the refrigerator at 4°C to promote solidification. After the low-melting agarose had solidified, the mixture was transferred into an incubator at 37°C for inversion culture until clear blue spots appeared.
6. The blue spots (recombinant vaccinia virus rTV-αCD47-αCD3-BITE) were picked to 500 μL of complete medium, which was repeatedly freezing and thawing at -80°C for more than three times to release as much virus as possible.
7. 143TK-cells were plated in 6-well plates at approximately $1 \times 10^6$ per well. Incubation was performed for approximately 24 h until the cells adhered and spreaded across the entire floor.
8. The blue spots in the EP tube were repeatedly blown and dispersed completely.

9. The complete medium was changed to maintenance medium and then the virus solution containing the blue spots was added and incubated in an incubator at 37°C for 3-4 h.

10. Adding screening pressure: the working concentration of BrdU was 50 $\mu$g/mL, the cells were incubated in an incubator at 37°C for about 48 h, and plated according to the formation of virus spots. This purification process needed to be performed at least five times.

11. A small sample of recombinant vaccinia virus was then amplified, and 143TK-cells were plated in six-well plates at 1 x 10^6 cells per well, using approximately 100% of the bottom area of the plate.

12. The medium in the wells was changed to 2 mL maintenance medium prior to seeding. The purified virus solution containing the blue spots was repeatedly blasted until the blue spots were dispersed. About 100 $\mu$L of virus solution was added to each well, incubated at 37°C for about 48 h, and the samples were collected according to the formation of virus spots.

13. Collecting sample: the culture medium supernatant in the wells was carefully aspirated by 1 mL. The cells were thoroughly blown down with the remaining 1 mL of medium and taken up in EP tubes for subsequent genomic extraction and amplification as virus seed.

### 4.3 Amplification and purification of $\alpha$CD47-$\alpha$CD3-BITE recombinant vaccinia virus

1. VERO cell plating: 10 cm dishes, about $5 \times 10^6$ cells per dish, it was appropriate to ensure that the cell density reached 100% when inoculating the vaccinia virus on the next day.

2. Before virus inoculation, complete medium was replaced with 8 mL maintenance medium (DMEM medium + 2% FBS + 1% PS), and virus was inoculated into cells in maintenance medium, with the inoculum size of about 0.02 MOI (MOI = virus PFU/cell number), which was continued to culture in an incubator containing 5% $CO_2$ at 37°C for about 48 h, and the samples was collected according to the formation of virus spots.

3. Collecting vaccinia virus: 8 mL culture medium in the dish was discarded, 2 mL maintenance culture medium was taken to blow down the remaining cells, and the virus was collected into a 15 mL centrifuge tube.

4. After cryopreservation for 24 h, the obtained virus solution was repeatedly frozen and thawed twice, density gradient centrifugation was performed with 36% sucrose solution, and centrifuged at 16000 g at 4°C for 90 min, the supernatant was carefully discarded, the virus precipitate in the centrifuge tube was dissolved with PBS buffer, dispensed and stored at -80°C, and the virus titer was to be determined.

### 4.4 Titer determination of recombinant vaccinia virus $\alpha$CD47-$\alpha$CD3-BITE

1. Preparation of 143TK cells: 143TK- cells were plated in a 24-well plate, with about $2 \times 10^5$ cells per well, and the cell density should reach 100% of the bottom area of the 24-well plate when used.

2. The virus was diluted, the vaccinia virus solution was diluted with maintenance medium. Starting from 1:100, 10-fold dilution was made, and the final volume was 1100 $\mu$L.

3. The complete medium in the 24-well plate was discarded, 500 $\mu$L of diluted virus solution was taken and added into the well, and two duplicate wells were made. Incubation was performed at 37°C in a 5% $CO_2$ incubator for about 48 h, and the plating time was determined according to the formation of virus plaque.

4. Plating method: 8 mL of plating medium containing 2 x DMEM medium + 4% FBS + 2% PS and 8 mL of low-melting agarose thawed in a boiling water bath and placed in 37°C water bath were prepared, the two were mixed, and then X-gal was added to the mixture, with the final concentration of 200 $\mu$g/mL, for later use.

5. The supernatant was aspirated from the 24-well plate. The plating mixture in step 4 was immediately added to a 24-well plate at 500 $\mu$L per well. Then it was carefully put into a refrigerator at 4°C to promote solidification, and after the low-melting-point agarose had solidified, the same was transferred into an incubator at 37°C for inversion culture until clear blue spots appeared.

6. Virus plaque counting: first, whether the number of viral plaques decreases in a ten-fold trend was observed, then the number of blue spots with only one digit in two duplicate wells of seed virus was counted. The sum of the values of blue spots obtained in the two wells multiplied the reciprocal value of the corresponding dilution of the well to obtain the virus titer in 1 mL.

### 4.5 Verification of expression of recombinant vaccinia virus rTV-$\alpha$CD47-$\alpha$CD3-BITE

1. Collecting virus supernatant: 10 cm culture dish was taken, and $5 \times 10^6$ VERO cells/dish were inoculated, so as to ensure that the cell density reached 100% when inoculating the vaccinia virus on the next day. Before virus inoculation, complete medium was replaced with 8 mL maintenance medium (DMEM medium + 2% FBS + 1% PS); then viruses were added, with the inoculum size of about 0.02 MOI (MOI = virus PFU/cell number), incubated in a 37°C 5% $CO_2$ incubator for 48 h; the cell supernatant was collected according to the formation of virus plaque, centrifuged at 10000 g for 5 min, and the supernatant was taken into a new centrifuge tube.

2. Subjecting the virus supernatant to His tag protein purification: the purification was performed manually using a syringe and the packed nickel beads were Ni-NTA Agarose (QIAGEN, Cat. No. 30210); after the completion of column loading, non-specifically adsorbed proteins were eluted with 5 nM and 10 nM imidazole (dissolved in 50 mM $NaH_2PO_4 \cdot 2H_2O$, pH 8.0, 300 mM NaCl) at the natural flow rate, and the filtrate was collected; the target protein was then eluted with 20 nM, 100 nM, 200 nM, 500 nM imidazole (dissolved in 50 mM $NaH_2PO_4 \cdot 2H_2O$, pH 8.0, 300 mM NaCl) and the corresponding filtrate was collected.

3. 30 μL of the filtrate was taken to prepare samples, 10% SDS-PAGE detection was performed and the results were shown in FIG. 4B. Samples were loaded sequentially from 5 mM imidazole eluate to 500 mM imidazole eluate. As shown in FIG. 4C, the protein of interest appeared in the 200 nM eluate, with a band size of around 54 kD.

Example 5: Affinity detection of recombinant vaccinia virus rTV-αCD47-αCD3-BITE

1. Collecting virus supernatant: 10 cm culture dish was taken, and $5 \times 10^6$ VERO cells/dish were inoculated, so as to ensure that the cell density reached 100% when inoculating the vaccinia virus on the next day. Before virus inoculation, the complete medium was replaced with 8 mL maintenance medium (DMEM medium + 2% FBS + 1% PS); then virus was added, with the inoculation amount of about 0.02 MOI (MOI = virus PFU/cell number), and at the same time, the wild-type virus control was set; incubation was performed in a 37°C 5% $CO_2$ incubator for 48 h; the cell supernatant was collected according to the formation of virus plaque, centrifuged at 10000 g for 5 min, and the supernatant was taken into a new centrifuge tube for His tag protein purification.

2. His tag protein purification was performed by the method as described above.

3. Preparation of CD47-A549 cells: $2 \times 10^6$ cells were divided into two EP tubes, centrifuged at 800 g for 3 min and the supernatant was discarded.

4. Wash was conducted twice with 1 mL of pre-chilled wash ($1 \times$ PBS + 2% FBS), centrifugation was performed at 800 g for 3 min and the supernatant was discarded.

5. An appropriate amount of purified protein was incubated with CD47-A549 cells at room temperature for 15 min.

6. Wash was conducted twice with 1 mL of pre-chilled wash, centrifugation was performed at 800 g for 3 min and the supernatant was discarded.

7. 0.1 μL of PE-labeled anti-His antibody was added to each sample for 15 min at room temperature.

8. Wash was conducted twice with 1 mL of pre-chilled wash, centrifugation was performed at 800 g for 3 min and the supernatant was discarded.

9. Each sample was resuspended in 200 μL of wash solution and affinity was determined by flow cytometry.

The result in FIG. 5 showed that cell supernatant expressed by vaccinia virus recombined with αCD47-αCD3-BITE can bind to CD47 with a positive rate of 85.9% compared to wild-type vaccinia virus. The positive clone was picked for the passage stability test, and the virus strain with strong passage stability and high expression of the target protein was selected and deposited, which was named rTV-αCD47-αCD3-BITE, with the deposit accession number: CCTCC NO: V202081, and deposited on January 2, 2021 at China Center for Type Culture Collection, address: Wuhan University, Wuhan, China.

Example 6: Effect of recombinant vaccinia virus with αCD47-αCD3-BITE in vitro against lung cancer

1. 143TK-Cell Plating: 143TK-cells were plated in a 6-well plate, with about $1 \times 10^6$ cells per well, and the cell density should reach 100% of the bottom area of the 6-well plate when used.

2. The complete medium was changed to 2 mL maintenance medium per well (DMEM medium + 2% FBS + 1% PS) prior to virus inoculation and the cells were inoculated with the virus at 0.02 MOI. Cells were incubated at 37°C in a 5% $CO_2$ incubator for about 48 h, and when to collect the supernatant was determined according to the virus plaque formation. At the same time, wild vaccinia virus was used as control.

3. 100 μL of the supernatant was taken separately for subsequent A549 in vitro killing assay.

4. The in vitro killing assay of A549 cells was performed according to the instructions of kit (Dojindo, Cat. No. CK17); 100 μL of resuspended A549 cells was pipetted into a 96-well plate with $1 \times 10^4$ cells per well, and incubated at 37°C, 5% $CO_2$ overnight.

5. The next day, the supernatant was added, cells were incubated at 37°C, 5% $CO_2$ for 1 h, and then T cells were added at an effect-target ratio of 5:1, and cells were continued to be incubated for 4 h at 37°C, 5% $CO_2$.

6. Following the addition of 20 $\mu$L of lysis buffer to the high control wells, 20 $\mu$L of the medium was added to the low control wells and background wells, and cells were incubated for 30 min at 37°C, 5% $CO_2$.

7. 100 $\mu$L of supernatant was pipetted from each well into a new 96-well plate.

8. After adding 100 $\mu$L of the developing solution to each well, it was reacted for 5 min at room temperature in the dark.

9. Finally, the assay was stopped by adding 50 $\mu$L stop solution to each well, the absorbance at 490 nm was immediately measured with a microplate reader.

Results were as shown in FIG. 6, recombinant vaccinia virus loaded with $\alpha$CD47-$\alpha$CD3-BITE significantly increased the killing of A549 cells in vitro by as much as 4-fold compared to the blank control group.

In conclusion, $\alpha$CD47-$\alpha$CD3-BITE protein combined with T cells had very strong killing activity on tumor cells, and the recombinant vaccinia virus rTV-$\alpha$CD47-$\alpha$CD3-BITE prepared as oncolytic virus can also significantly control a variety of solid tumors such as human lung cancer, which had very high application value for the treatment of tumors and was simple to prepare, thereby being convenient for large-scale preparation and promotion.

Example 7: In vitro anti-human ovarian adenocarcinoma cell effect of T cells mediated by $\alpha$CD47-$\alpha$CD3-BITE of different structural formulae

In order to prove the anticancer effect of $\alpha$CD47-$\alpha$CD3-BITE proteins with different structures, the applicant respectively designed the following BITE proteins:

BITE1: $VL_{\alpha CD47}$-L-$VH_{\alpha CD3}$-L -$VL_{\alpha CD3}$-L -$VH_{\alpha CD47}$ the $VH_{\alpha CD47}$ was as shown in SEQ ID NO: 19, the $VL_{\alpha CD47}$ was as shown in SEQ ID NO: 21, the $VH_{\alpha CD3}$ was as shown in SEQ ID NO: 25, the $VL_{\alpha CD3}$ was as shown in SEQ ID NO: 26, and the L was GGGGSGGGGSGGGGS.

BITE2: $VH_{\alpha CD47}$-L-$VH_{\alpha CD3}$-L-$VL_{\alpha CD3}$-L-$VL_{\alpha CD47}$ the $VH_{\alpha CD47}$ was as shown in SEQ ID NO: 19, the $VL_{\alpha CD47}$ was as shown in SEQ ID NO: 21, the $VH_{\alpha CD3}$ was as shown in SEQ ID NO: 25, the $VL_{\alpha CD3}$ was as shown in SEQ ID NO: 26, and the L was GGGGSGGGGSGGGGS.

BITE3: $VH_{\alpha CD47}$-L-$VL_{\alpha CD47}$-L-$VH_{\alpha CD3}$-L-$VL_{\alpha CD3}$ the $VH_{\alpha CD47}$ was as shown in SEQ ID NO: 19, the $VL_{\alpha CD47}$ was as shown in SEQ ID NO: 21, the $VH_{\alpha CD3}$ was as shown in SEQ ID NO: 25, the $VL_{\alpha CD3}$ was as shown in SEQ ID NO: 26; one linker peptide was GGGGSGGGGSGGGGS.

BITE4: Like BITE1, only the linker peptide was replaced with GGGGGG;

BITES: Like BITE1, the $VH_{\alpha CD3}$ was as shown in SEQ ID NO: 27 and $VL_{\alpha CD3}$ was as shown in SEQ ID NO: 28.

The example was the same as Examples 2 and 3 and the results were shown in FIG. 7. $VL_{\alpha CD47}$-L-$VH_{\alpha CD3}$-L -$VL_{\alpha CDS}$-L -$VH_{\alpha CD47}$, in which L was $(G_4S)_3$, was the optimal design of $\alpha$CD47-$\alpha$CD3-BITE, which can mediate strong killing function against tumor cells; when the antibody clone types of CD47 or CD3 (such as clone No. SP34) was changed, the BiTE can still have strong killing function; VH$\alpha$CD47-L-VH$\alpha$CD3-L-VL$\alpha$CD3-L-VL$\alpha$CD47, where L was $(G_4S)_3$, was a suboptimal design for $\alpha$CD47-$\alpha$CD3-BITE, but still had tumor killing activity similar to the control antibody.

The above-described embodiments are exemplary and are not to be construed as limiting the present invention, and variations, modifications, substitutions, and alterations to the above-described embodiments may be made by those of ordinary skill in the art within the scope of the present invention.

## Claims

1. An $\alpha$CD47 and $\alpha$CD3 bispecific T-cell engager $\alpha$CD47-$\alpha$CD3 BiTE, comprising a fusion protein of any one of the following formulae:

$$VL_{\alpha CD47}\text{-L-}VH_{\alpha CD3}\text{-L-}VL_{\alpha CD3}\text{-L-}VH_{\alpha CD47};$$

or

$$VH_{\alpha CD47}\text{-L-}VH_{\alpha CD3}\text{-L-}VL_{\alpha CD3}\text{-L-}VL_{\alpha CD47}$$

wherein L represents a linker peptide; wherein the $VH_{\alpha CD47}$ is the heavy chain variable region of a CD47 antibody comprising the following three complementary determining regions:

(i) VH CDR1 consisting of the following sequence: SEQ ID NO: 1, or a sequence having one or several amino acid substitutions, deletions, or additions compared thereto,
(ii) VH CDR2 consisting of the following sequence: SEQ ID NO: 2, or a sequence having one or several amino acid substitutions, deletions, or additions compared thereto, and
(iii) VH CDR3 consisting of the following sequence: SEQ ID NO: 3, or a sequence having one or several amino acid substitutions, deletions, or additions compared thereto;

preferably, the substitution of any one of (i)-(iii) is a conservative substitution; preferably, the VH$_{\alpha CD47}$ comprises VH CDR1 as shown in SEQ ID NO: 1, VH CDR2 as shown in SEQ ID NO: 2, VH CDR3 as shown in SEQ ID NO: 3; wherein the VL$_{\alpha CD47}$ is the light chain variable region of a CD47 antibody comprising the following three complementary determining regions:

> (iv) VL CDR1 consisting of the following sequence: a sequence as shown in any one of SEQ ID NO: 4, SEQ ID NO: 7, SEQ ID NO: 10, SEQ ID NO: 13, or SEQ ID NO: 16, or having one or several amino acid substitutions, deletions, or additions compared thereto,
> (v) VL CDR2 consisting of the following sequence: a sequence as shown in any one of SEQ ID NO: 5, SEQ ID NO: 8, SEQ ID NO: 11, SEQ ID NO: 14, or SEQ ID NO: 17, or having one or several amino acid substitutions, deletions, or additions compared thereto, and
> (vi) VL CDR3 consisting of the following sequence: a sequence as shown in any one of SEQ ID NO: 6, SEQ ID NO: 9, SEQ ID NO: 12, SEQ ID NO: 15, or SEQ ID NO: 18, or having one or several amino acid substitutions, deletions, or additions compared thereto;
> preferably, the substitution of any one of (iv)-(vi) is a conservative substitution;
> preferably, the VL$_{\alpha CD47}$ comprises VL CDR1 as shown in SEQ ID NO: 4, VL CDR2 as shown in SEQ ID NO: 5, and VL CDR3 as shown in SEQ ID NO: 6;
> or the VL$_{\alpha CD47}$ comprises VL CDR1 as shown in SEQ ID NO: 7, VL CDR2 as shown in SEQ ID NO: 8, and VL CDR3 as shown in SEQ ID NO: 9;
> or the VL$_{\alpha CD47}$ comprises VL CDR1 as shown in SEQ ID NO: 10, VL CDR2 as shown in SEQ ID NO: 11, and VL CDR3 as shown in SEQ ID NO: 12;
> or the VL$_{\alpha CD47}$ comprises VL CDR1 as shown in SEQ ID NO: 13, VL CDR2 as shown in SEQ ID NO: 14, and VL CDR3 as shown in SEQ ID NO: 15;
> or the VL$_{\alpha CD47}$ comprises VL CDR1 as shown in SEQ ID NO: 16, VL CDR2 as shown in SEQ ID NO: 17, and VL CDR3 as shown in SEQ ID NO: 18.

2. The bispecific T-cell engager of claim 1, wherein the VH$_{\alpha CD47}$ comprises three CDRs contained in the heavy chain variable region as shown in SEQ ID NO: 19;

> preferably, the VL$_{\alpha CD47}$ comprises three CDRs contained in the light chain variable region as shown in any one of SEQ ID NOs: 20-24;
> preferably, the three CDRs contained in the heavy chain variable region and/or the three CDRs contained in the light chain variable region are defined by the Kabat, Chothia, or IMGT numbering system.

3. The bispecific T-cell engager of claim 1 or 2, wherein the VH$_{\alpha CD47}$ comprises an amino acid sequence selected from the group consisting of:

> (i) a sequence as shown in SEQ ID NO: 19;
> (ii) a sequence having one or several amino acid substitutions, deletions, or additions compared to the sequence as shown in SEQ ID NO: 19; or
> (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 19;
> and/or,
> the VL$_{\alpha CD47}$ comprises an amino acid sequence selected from the group consisting of:
>
>> (iv) a sequence as shown in any one of SEQ ID NOs: 20-24;
>> (v) a sequence having one or several amino acid substitutions, deletions, or additions compared to the sequence as shown in any one of SEQ ID NOs: 20-24; or
>> (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in any one of SEQ ID NOs: 20-24;
>> preferably, the substitution in (ii) or (v) is a conservative substitution.

4. The bispecific T-cell engager of any one of claims 1 to 3, wherein the VH$_{\alpha CD3}$ comprises an amino acid sequence selected from the group consisting of:

> (i) a sequence as shown in SEQ ID NO: 25 or 27;

(ii) a sequence having one or several amino acid substitutions, deletions, or additions compared to the sequence as shown in SEQ ID NO: 25 or 27; or

(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 25 or 27;

and/or,

the VL$_{\alpha CD3}$ comprises an amino acid sequence selected from the group consisting of:

(iv) a sequence as shown in SEQ ID NO: 26 or 28;

(v) a sequence having one or several amino acid substitutions, deletions, or additions compared to the sequence as shown in SEQ ID NO: 26 or 28; or

(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as shown in SEQ ID NO: 26 or 28;

preferably, the substitution in (ii) or (v) is a conservative substitution.

5. The bispecific T-cell engager of any one of claims 1 to 4, wherein the VH$_{\alpha CD47}$, VH$_{\alpha CD3}$, VL$_{\alpha CD47}$, and VL$_{\alpha CD3}$ are linked by a linker peptide;

preferably, the VH$_{\alpha CD47}$, VH$_{\alpha CDS}$, VL$_{\alpha CD47}$, and VL$_{\alpha CD3}$ are linked by one, two, or three linker peptides;

more preferably, L is KESGSVSSEQ-LAQFRSLD, EGKSSGSGSESKST, GGGGGGG, GGGGGGGG or (GGGGS)$_n$; further preferably, n is an integer from 1 to 5, most preferably n is 3;

preferably, the bispecific T-cell engager comprises a fusion protein of the following formula:

$$VH_{\alpha CD47}\text{-}L\text{-}VH_{\alpha CD3}\text{-}L\text{-}VL_{\alpha CD3}\text{-}L\text{-}VL_{\alpha CD47}$$

wherein the VH$_{\alpha CD47}$ is as shown in SEQ ID NO: 19, the VL$_{\alpha CD47}$ is as shown in SEQ ID NO: 21 or 24, the VH$_{\alpha CD3}$ is as shown in SEQ ID NO: 25, VL$_{\alpha CD3}$ is as shown in SEQ ID NO: 26, and the L is GGGGSGGGGSGGGGS.

6. An isolated nucleic acid molecule, encoding the bispecific T-cell engager of any one of claims 1 to 5;

preferably, the nucleic acid molecule comprising a nucleic acid sequence as shown in any one of SEQ ID NOs: 31-35, the nucleic acid sequence being the coding sequence for VL$_{\alpha CD47}$;

preferably, the nucleic acid molecule comprising a nucleic acid sequence as shown in SEQ ID NO: 36, the nucleic acid sequence being the coding sequence for VH$_{\alpha CD47}$.

7. An expression framework for the bispecific T-cell engager of any one of claims 1 to 5: 5 '-E1-E2-E3 -E4-E5 -E6-E7-3' wherein:

E1 is a CMV enhancer or/and other cis-acting elements, preferably comprises the nucleic acid sequence as shown in SEQ ID NO: 37;

E2 is a recombinantly expressed promoter, preferably a CMV promoter, more preferably comprises the nucleic acid sequence as shown in SEQ ID NO: 38;

E3 is a 5' untranslated region, optionally comprises no or one intron sequence, optionally comprises one or more restriction enzyme sites, preferably comprises the nucleic acid sequence as shown in SEQ ID NO: 39;

E4 is a coding nucleotide sequence of the signal peptide of the BiTE protein; preferably, the signal peptide is derived from a human or mouse signal peptide; preferably E4 comprises the nucleic acid sequence as shown in SEQ ID NO: 40;

E5 is a coding nucleotide sequence of the bispecific T-cell engager of any one of claims 1 to 5;

E6 is a 3' untranslated region, optionally comprises one or more restriction enzyme sites, preferably comprises the nucleic acid sequence as shown in SEQ ID NO: 41;

E7 is an SV40 transcription termination signal region, preferably comprises the nucleic acid sequence as shown in SEQ ID NO: 42.

8. A recombinant oncolytic virus, the recombinant oncolytic virus being operably inserted with or comprising the expression framework of claim 7; wherein

preferably, the expression framework is located in the thymidine kinase (TK) region of the recombinant oncolytic virus;

preferably, the expression framework can be expressed alone, or be fusion expressed with other genes or fragments;

preferably, the recombinant oncolytic virus further comprises gene coding sequences for other immunomodulatory factors, more preferably, the other immunomodulatory factors include but are not limited to IL-1, IL-2, IL-3, IL-7, IL-11, IL-12, IL-15, IL-17, IL-18, IL-21, IL-33, IL-35, IL-37, GM-CSF, IFN-$\alpha$, IFN-$\beta$, IFN-$\gamma$, anti-PD-1/PD-L1 antibody, anti-CTLA-4 antibody, anti-Lag-3 antibody, anti-TIGIT antibody, or anti-Tim-3 antibody; or

the recombinant oncolytic virus further comprises a gene coding sequence of an apoptosis- and pyroptosis-related protein, preferably the apoptosis- and pyroptosis-related protein is selected from apoptosis-related factor 1, interleukin-1 β converting enzyme, Bcl-2 protein, Fas/-APO-1, p53, myc, ataxia telangiectasia mutant gene, gasdermin D, or gasdermin E; or the recombinant oncolytic virus further comprises a small RNA targeting immunomodulatory genes, apoptosis and pyroptosis genes.

9. The recombinant oncolytic virus of claim 8, wherein the viral backbone of the oncolytic virus is derived from a modified or engineered vaccinia virus Tian Tan strain, vaccinia virus New York strain, vaccinia virus Copenhagen strain, vaccinia virus canary strain, vaccinia virus Ankara strain, adenovirus, adeno-associated virus, herpes simplex virus, varicella-zoster virus, respiratory syncytial virus, Semliki forest virus, EB virus, cytomegalovirus, human Herpesvirus type 6, smallpox virus, vaccinia virus, molluscum contagiosum virus, sheep aphthovirus, reovirus, rotavirus, enterovirus, Seneca virus, poliovirus, coxsackie virus, rhinovirus, hepatitis A virus, foot and mouth disease virus, togavirus, alphavirus, Semiliki forest virus, eastern equine encephalitis virus, Sindbis virus, rubella virus, coronavirus, flavivirus, hepatitis C virus, Japanese encephalitis virus, St. Louis encephalitis virus, Murray Valley fever virus, yellow fever virus, West Nile virus, zika virus, dengue virus, Ebola virus, Marburg virus, arenavirus, Lassa fever virus, lymphocytic choriomeningitis virus, Pichinde virus, Junin virus, Machupo virus, Hantavirus, Rift Valley fever virus, Paramyxovirus, human parainfluenza virus, Mumps virus, simian virus 5, measles virus, vesicular stomatitis virus, rabies virus, orthomyxovirus, influenza A virus, influenza B virus, influenza C virus, hepatitis D virus, simian immunodeficiency virus, human immunodeficiency virus type 1 and human immunodeficiency virus type 2, Rous sarcoma virus, human T-cell leukemia virus type 1, simian foamy virus, hepatitis B virus, hepatitis E virus, human papilloma virus, or polyomavirus; preferably, the oncolytic viral backbone is an intracellular maturation virus, an intracellular packaging virus, a cell-associated packaging virus, or an extracellular packaging virus.

10. A recombinant vaccinia virus Tian Tan strain rTV-αCD47-αCD3-BITE, with the deposit accession number of CCTCC NO: V202081.

11. A method for preparing the recombinant oncolytic virus of any one of claims 8 to 10, comprising the steps of:

1) synthesizing an expression framework of the bispecific T-cell engager BiTE of claim 7;
2) subcloning the expression framework obtained in step 1) into a shuttle plasmid of an oncolytic virus to construct a recombinant plasmid vector;
3) transfecting the recombinant plasmid vector obtained in step 2) into an oncolytic virus, and screening the same so as to obtain a recombinant oncolytic virus;

optionally, culturing the obtained recombinant oncolytic virus;
preferably, the method comprising the steps of:

1) synthesizing an expression framework of the bispecific T-cell engager αCD47-αCD3-BITE, which comprises a nucleic acid sequence as shown in any one of SEQ ID NOs: 31-42;
2) subcloning the synthesized expression framework into the TK region of the vaccinia virus shuttle plasmid (pSC65) to construct the recombinant plasmid pSC65-αCD47-αCD3-BITE;
3) transfecting the pSC65-αCD47-αCD3-BITE plasmid into TK143-cells which have been infected with wild-type vaccinia virus by means of gene homologous recombination, and homologously recombing the two to produce recombinant vaccinia virus rTV-αCD47-αCD3-BITE; screening the same to obtain a recombinant oncolytic vaccinia virus wherein the TK region comprises a coding sequence of αCD47-αCD3-BITE;

preferably, the expression framework of the bispecific T-cell engager αCD47-αCD3-BITE is controlled by the early/late promoter p7.5 of vaccinia virus.

12. Use of the bispecific T-cell engager of any one of claims 1 to 5 or the recombinant oncolytic virus of any one of claims 8 to 10 for the preparation of an anti-tumor medicament;
preferably, the tumor being selected from the group consisting of B-cell lymphoma; T-cell lymphoma; melanoma; prostate cancer; renal cell carcinoma; sarcoma; glioma, such as high-grade glioma; blastoma, such as neuroblastoma; osteosarcoma; plasmacytoma; histiocytoma; pancreatic cancer; breast cancer; lung cancer, such as small cell lung cancer and non-small cell lung cancer; gastric cancer; liver cancer; colon cancer; rectal cancer; esophageal cancer; colorectal cancer; hematopoietic cancer; testicular cancer; cervical cancer; ovarian cancer;

bladder cancer; squamous cell cancer; adenocarcinoma; AIDS-related lymphoma; bladder cancer; brain cancer; nervous system cancer; head and neck cancer; head and neck squamous cell carcinoma; Hodgkin's lymphoma; non-Hodgkin's lymphoma; or hematological tumorigenesis disease.

13. A method of treating a tumor, comprising administering to a subject in need thereof a therapeutically effective amount of the bispecific T-cell engager of any one of claims 1 to 5 or the recombinant oncolytic virus of any one of claims 8 to 10.

FIG. 1A

FIG. 1B

## SK-OV3

FIG. 2

## NCI-H292

FIG. 3

FIG. 4A

FIG. 4B

FIG. 4C

TV                                    rTV-αCD47-αCD3-BITE

FIG. 5

FIG. 6

## NCI-H292

FIG. 7A

## SK-OV3

FIG. 7B

**EP 4 458 862 A1**

## INTERNATIONAL SEARCH REPORT

| International application No. |
|---|
| **PCT/CN2021/141634** |

### A. CLASSIFICATION OF SUBJECT MATTER

C07K 19/00(2006.01)i; C12N 7/01(2006.01)i; C12N 15/62(2006.01)i; C12N 15/863(2006.01)i; A61K 35/76(2015.01)i; A61K 35/768(2015.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

C07K, C12N, A61K, A61P

Documentation searched other than minimum documentation to the extent that such documents are included in the fields searched

Electronic data base consulted during the international search (name of data base and, where practicable, search terms used)

Databases: CNTXT, WPABSC, ENTXT, WPABS, CJFD, DWPI, ENTXTC, VEN, CNKI, PubMed, Genbank, Web of Science, Science Direct, 百度, Baidu; Search terms: 双特异, 双功能, 抗体, 溶瘤病毒, 痘苗病毒, CD47, CD3, bifunction+, bispecific t cell engager, t cell engager, BiTE, vaccinia, 上海鑫湾生物科技有限公司, SEQ ID NO: 1-36

### C. DOCUMENTS CONSIDERED TO BE RELEVANT

| Category* | Citation of document, with indication, where appropriate, of the relevant passages | Relevant to claim No. |
|---|---|---|
| E | CN 114249836 A (SHANGHAI XINWAN BIOTECHNOLOGY CO., LTD.) 29 March 2022 (2022-03-29)<br>see claims 1-12 | 1-12 |
| A | CN 111138546 A (ANYUAN PHARMACEUTICAL TECHNOLOGY (SHANGHAI) CO., LTD.) 12 May 2020 (2020-05-12)<br>see embodiment 6, table 6-2 | 1-12 |
| A | CN 111448213 A (SEATTLE GENETICS INC.) 24 July 2020 (2020-07-24)<br>see description, paragraphs 88-89 | 1-12 |
| A | WO 2018049261 A1 (ICELLHEALTH CONSULTING LLC.) 15 March 2018 (2018-03-15)<br>see claims 1-39 | 1-12 |
| A | CN 103665165 A (JIANGSU KUANGYA BIOLOG MEDICAL SCIENCE & TECHNOLOGY CO., LTD.) 26 March 2014 (2014-03-26)<br>see claims 1-10, figure 1 | 1-12 |

☑ Further documents are listed in the continuation of Box C.  ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 August 2022** | **25 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/141634**

**C.    DOCUMENTS CONSIDERED TO BE RELEVANT**

| Category* | Citation of document, with indication, where appropriate, of the relevant passages | Relevant to claim No. |
|---|---|---|
| A | LIU, Shumin et al. "A Long Acting Bi-Specific T Cell Engager Differentially Targeting CD47 Positive Malignant Cells but not CD47 Expressing Healthy Cells" *Cancer Research*, Vol. 81, No. (13), 21 May 2021 (2021-05-21), see passage 1868, abstract | 1-12 |

Form PCT/ISA/210 (second sheet) (January 2015)

## INTERNATIONAL SEARCH REPORT

International application No.

**PCT/CN2021/141634**

**Box No. I**    **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

     a.  ☑ forming part of the international application as filed:

          ☑ in the form of an Annex C/ST.25 text file.

          ☐ on paper or in the form of an image file.

     b.  ☐ furnished together with the international application under PCT Rule 13*ter*.1(a) for the purposes of international search only in the form of an Annex C/ST.25 text file.

     c.  ☐ furnished subsequent to the international filing date for the purposes of international search only:

          ☐ in the form of an Annex C/ST.25 text file (Rule 13*ter*.1(a)).

          ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

International application No.

**PCT/CN2021/141634**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **13**
because they relate to subject matter not required to be searched by this Authority, namely:

    [1]  A method for treating a living human or animal body, which falls within the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

| International application No. |
| --- |
| **PCT/CN2021/141634** |

| Patent document cited in search report | | | Publication date (day/month/year) | Patent family member(s) | | | Publication date (day/month/year) |
| --- | --- | --- | --- | --- | --- | --- | --- |
| CN | 114249836 | A | 29 March 2022 | None | | | |
| CN | 111138546 | A | 12 May 2020 | EP | 3875485 | A1 | 08 September 2021 |
| | | | | AU | 2019370758 | A1 | 10 June 2021 |
| | | | | EP | 3875479 | A1 | 08 September 2021 |
| | | | | US | 2021371526 | A1 | 02 December 2021 |
| | | | | US | 2022002431 | A1 | 06 January 2022 |
| | | | | CN | 111138544 | A | 12 May 2020 |
| | | | | CN | 112996817 | A | 18 June 2021 |
| | | | | CN | 112996807 | A | 18 June 2021 |
| | | | | CN | 111138545 | A | 12 May 2020 |
| | | | | WO | 2020088608 | A1 | 07 May 2020 |
| | | | | KR | 20210087472 | A | 12 July 2021 |
| | | | | EP | 3889179 | A1 | 06 October 2021 |
| | | | | WO | 2020088437 | A1 | 07 May 2020 |
| | | | | JP | 2022512997 | A | 07 February 2022 |
| | | | | CL | 2021001143 | A1 | 22 October 2021 |
| | | | | WO | 2020088164 | A1 | 07 May 2020 |
| | | | | CA | 3118238 | A1 | 07 May 2020 |
| | | | | CN | 111138542 | A | 12 May 2020 |
| | | | | KR | 20210089697 | A | 16 July 2021 |
| | | | | EP | 3875489 | A1 | 08 September 2021 |
| | | | | CN | 112996810 | A | 18 June 2021 |
| | | | | AU | 2019370339 | A1 | 10 June 2021 |
| | | | | CN | 111138547 | A | 12 May 2020 |
| | | | | WO | 2020088403 | A1 | 07 May 2020 |
| | | | | US | 2022002407 | A1 | 06 January 2022 |
| | | | | CA | 3118397 | A1 | 07 May 2020 |
| | | | | EP | 3889174 | A1 | 06 October 2021 |
| | | | | WO | 2020088605 | A1 | 07 May 2020 |
| | | | | CO | 2021006970 | A2 | 30 July 2021 |
| | | | | CN | 112955461 | A | 11 June 2021 |
| | | | | PE | 20211867 | A1 | 21 September 2021 |
| | | | | JP | 2022512865 | A | 07 February 2022 |
| CN | 111448213 | A | 24 July 2020 | US | 2022135674 | A1 | 05 May 2022 |
| | | | | AR | 113862 | A1 | 17 June 2020 |
| | | | | JP | 2021503927 | A | 15 February 2021 |
| | | | | SG | 11202004864T | A | 29 June 2020 |
| | | | | AU | 2018375375 | A1 | 28 May 2020 |
| | | | | KR | 20200091901 | A | 31 July 2020 |
| | | | | BR | 112020009805 | A2 | 13 October 2020 |
| | | | | TW | 201925227 | A | 01 July 2019 |
| | | | | EA | 202091339 | A1 | 21 October 2020 |
| | | | | EP | 3717515 | A2 | 07 October 2020 |
| | | | | US | 2019185561 | A1 | 20 June 2019 |
| | | | | WO | 2019108733 | A2 | 06 June 2019 |
| | | | | MA | 51208 | A | 07 October 2020 |
| | | | | IL | 274519 | A | 30 June 2020 |
| | | | | CA | 3083946 | A1 | 06 June 2019 |
| WO | 2018049261 | A1 | 15 March 2018 | WO | 2018049248 | A1 | 15 March 2018 |
| | | | | TW | 201825674 | A | 16 July 2018 |

Form PCT/ISA/210 (patent family annex) (January 2015)

INTERNATIONAL SEARCH REPORT
Information on patent family members

International application No.

**PCT/CN2021/141634**

| Patent document cited in search report | | | Publication date (day/month/year) | Patent family member(s) | | | Publication date (day/month/year) |
|---|---|---|---|---|---|---|---|
| | | | | TW | 201825511 | A | 16 July 2018 |
| CN | 103665165 | A | 26 March 2014 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9017675 B2